# EUROPEAN PATENT APPLICATION

(11) **EP 4 806 385 A1**
(43) Date of publication of application: **16.09.2026**
(21) Application number: 25163817.7
(22) Date of filing: 14.03.2025
(51) Int. Cl.: A61C 5/62, B05C 17/005

(54) **APPLICATOR, APPLICATOR TIP AND STORING COMPONENT**

(71) Applicant: medmix Switzerland AG, 9469 Haag (Rheintal) (CH)
(72) Inventor: AICHER, Martin, 9469 Haag (CH); MÜLLER, Frank, 6800 Feldkirch (AT); BAYER, Martin, 96472 Rödental (DE)
(74) Representative: Manitz Finsterwald Patent- und Rechtsanwaltspartnerschaft mbB

(57) **Abstract**

An applicator includes an applicator tip and a storing component.

The applicator tip comprises a cavity, a rear end comprising an opening for allowing the storing component to be partially inserted into the cavity,
a front end, a side wall extending between the rear end and the front end, and a cannula extending through the front end, the cannula being partly located in the cavity. The storing component comprises a container for storing a fluid material and a front portion configured to be housed in the cavity of the applicator tip. A removable connection is provided between the cavity and the front portion of the storing component, said removable connection comprising at least an interference fit or a snap-fit, and a fluid-tight connection is provided between the cannula and the storing component for fluidically coupling the cannula to the container.

## Description

The present disclosure generally refers to an applicator, an applicator tip for an applicator and a storing component for an applicator.

Applicators are well known in the art and may be used for instance in a dispensing system to dispense a material on an area of application. As such, the applicator may include an applicator tip comprising a discharge device, such as a cannula. The applicator may include a storing component for storing the material. Exemplary materials are 1K materials, fluids, cremes, medical fluids, dental fluids, sealing materials, etching materials, adhesives, ointments, paints and the like. These materials may be in particular used in medical applications such as dental or surgical applications.

In such applications, it is desirable to dispense a precise amount of material. Furthermore, in such an applicator it is desirable that the material flows from the storing component to the discharge device provided on the applicator tip, avoiding leakages between the applicator tip and the storing component. Hence, it is one object of the invention to provide a dispenser that allows for precise material dispensing and avoids leakages between the applicator tip and the storing component.

These objects are satisfied by the subject matter of the independent claims.

According to a first aspect of the invention, an applicator comprises an applicator tip and a storing component. The applicator tip comprises a cavity defining a housing for the storing component, a rear end comprising an opening for allowing the storing component to be partially inserted into the cavity, a front end defining a bottom of the cavity, a side wall extending between the rear end and the front end, and a cannula extending through the front end between a first cannula end and a second cannula end, the first cannula end being located in the cavity. The storing component comprises a container for storing a fluid material and a front portion configured to be housed in the cavity of the applicator tip. A removable connection is provided between the cavity and the front portion of the storing component, said removable connection comprising at least an interference fit or a snap-fit. A fluid-tight connection is provided between the cannula and the storing component for fluidically coupling the cannula to the container.

The applicator according to present invention comprises an applicator tip and a storing component. In order to correctly dispense the fluid material that is stored the contain, the front portion of the storing component is housed in the cavity of the applicator tip. The coupling between the first protrusion and the first mating surface and the coupling between the second protrusion and the second mating surface provides a removable, but secure and stable connection between the applicator tip and the storing component. The connection between the applicator tip and the storing component further allows the fluid-tight connection between the cannula and the storing component for fluidically coupling the cannula to the container. This permits the dispensing of the fluid material without leakages. This is achieved with a reduced number of elements. Each of the applicator tip and the storing component may be formed as a single piece, for example by means of injection moulding. This improves efficiency of the manufacturing process.

The removable connection may comprise at least a first protrusion on one of the side wall of the applicator tip and the front portion of the storing component. The removable connection may further comprise at least a first mating surface on the other of the side wall of the applicator tip and the front portion of the storing component. The first protrusion and the first mating surface are configured and dimensioned for coupling to each other when the front portion of the storing component is housed in the cavity.

According to embodiments of the present invention, the removable connection comprises the first protrusion and at least a second protrusion on the side wall. The removable connection further comprises the first mating surface and at least a second mating surface on the front portion of the storing component. The second protrusion and the second mating surface are configured and dimensioned for coupling to each other when the front portion of the storing component is housed in the cavity. The first protrusion is closer to the front end than the second protrusion.

The first mating surface and the second mating surface may be provided as respective depressions on the front portion of the storing component. According to embodiments of the present invention, the first mating surface and the second mating surface are provided as channels or grooves or concave recesses or flattened portion along the front portion of the storing component. The first mating surface and the second mating surface may longitudinally oriented, i.e., parallel or substantially parallel to a longitudinal axis of the storing component, so that the first protrusion and the second protrusion are guided when the applicator tip is coupled to the front portion. This improves the handling of the applicator.

According to other embodiments of the present invention, the side wall comprises the first mating surface and the second mating surface, while the front portion comprises the first protrusion and the second protrusion.

Advantageously, the coupling between the applicator tip and the storing component is provided by a plurality of features, thus improving stability of the connection. This prevents leakage and ensures safe and accurate operation of the applicator.

According to embodiments of the present invention, the first protrusion and the first mating surface are configured and dimensioned for coupling to each other by means of a transition fit and the second protrusion and the second mating surface are configured and dimensioned are configured and dimensioned for coupling to each other by means of an interference fit. Advantageously, such a configuration provides a smooth but stable coupling between the applicator tip and the storing component. Additionally, the second mating surface may comprise an edge protruding from the second mating surface and interposed between the second protrusion and the first protrusion when the front portion of the storing component is housed in the cavity with the first protrusion and the first mating surface coupled to each other and the second protrusion and the second mating surface coupled to each other. Advantageously, this further improves the stability of the connection.

According to embodiments of the invention, the front portion may comprise a valve fluid-tightly coupled with the first end of the cannula of the applicator tip when the front portion of the storing component is housed in the cavity with the first protrusion and the first mating surface coupled to each other and the second protrusion and the second mating surface coupled to each other. Particularly, the valve may comprise a membrane which is impermeable to the fluid material inside the container, the membrane comprising an elastic opening for allowing the first end of the cannula of the applicator tip to be inserted in the container when the front portion of the storing component is housed in the applicator tip with the first protrusion and the first mating surface coupled to each other and the second protrusion and the second mating surface coupled to each other. The membrane may be for example provided as a disc of elastic material to be fixed along its edge to a membrane seat provided in the storing component.

Advantageously, the valve provides an effective connection between the cannula of the applicator tip and the container, thus ensuring accurate delivery of the fluid material from the container.

The removable connections between the applicator tip and the storing component are configured for allowing a smooth coupling, so that pressure equalization in the applicator tip is provided during attachment to or removal from the front portion. This avoids rapid pressure fluctuations, which could cause the valve to open. This is achieved, for example, by conveniently dimensioning the first mating surface and the second mating surface as longitudinal channels or grooves or concave recesses or flattened portion.

The opening on the membrane may comprise one or more cuts provided on the membrane. For example, two or more cuts may be provided, in particular crossing each other at a point of intersection and being angularly offset with respect to one another about an axis of the storing component. According to one exemplary embodiment, the membrane includes two cuts, perpendicularly arranged to each other. According to one exemplary embodiment, the membrane includes three cuts, angularly offset of 120° with respect to one another. The membrane may be convex towards an inside of the container. Alternatively, the membrane may be planar.

Advantageously, the valve provided in the storing component assures a correct coupling between the cannula of the applicator tip and the container, for providing accurate delivery of the fluid material from the container, at the same time avoiding or reducing leakages.

According to other embodiments of the invention, the cannula is partially inserted in the inner duct of the front portion, the cannula contacting with the inner duct to provide said fluid - tight connection. Advantageously, the such coupling between the cannula and the inner duct of the storing component assures also a correct coupling between the cannula of the applicator tip and the container, for providing accurate delivery of the fluid material from the container, at the same time avoiding or reducing leakages. According to such embodiments, the inner duct of the front portion may divergent from the orifice to the container, in particular the inner duct may be inclined of an angle comprised between 1° and 3° with respect to an axis of the inner duct. Further advantageously, the pressure of the fluid material inside the container acts against the cannula, which is forced back from the container and towards the orifice. The inclination of the inner duct, counteracts the fluid pressure. As a result, the fluid tightness of the contact between the cannula and the inner duct is increased.

According to yet other embodiments of the invention, the fluid-tight contact between the cannula and the orifice of the storing component is provided between an external chamfer of the cannula and the orifice. The dimension of the end of the cannula allows only a portion of the chamfer to be inserted in the inner duct, thus providing the fluid -tight coupling with the inner duct.

According to other embodiments of the invention, a movable piston is provided in the container for dispensing the fluid material, the piston comprising at least a sealing lip or gasket for providing a seal between the piston and the container, the piston being integrally formed in one piece with the sealing lip or gasket. Similarly to the applicator tip and the storing component, the piston may be formed as a single piece, for example by means of injection moulding. This further improves efficiency of the manufacturing process.

According to a further aspect of the invention this relates to a piston that can be provided in the container for dispensing the fluid material, the piston comprising at least a sealing lip or gasket for providing a seal between the piston and the container, the piston being integrally formed in one piece with the sealing lip or gasket. The piston may be formed as a single piece, for example by means of injection moulding. The applicator may be provided as an assembly comprising three main elements: an applicator tip, a storing component and a piston. Each of the three components may be formed as a single piece, for example by means of injection moulding This further improves efficiency of the manufacturing process.

According to a second aspect of the invention, an applicator tip for an applicator, in particular for the applicator above described, is provided. The applicator tip comprises a cavity defining a housing for the storing component of the applicator, a rear end comprising an opening for allowing the storing component to be partially inserted into the cavity, a front end defining a bottom of the cavity, a side wall extending between the rear end and the front end, the side wall comprising an inner surface delimiting the cavity, and a cannula extending through the front end between a first cannula end and a second cannula end, the first cannula end being located in the cavity. The side wall comprises at least one connection element for providing a removable connection between the cavity and the front portion of the storing component, said removable connection comprising at least an interference fit or a snap-fit.

The applicator tip may include any of the features above described with reference to the applicator tip of the applicator according to the first aspect of the invention.

In particular, the connection elements may include a first protrusion and a second protrusion, respectively configured and dimensioned for coupling to a first and a second mating surface provided on the storing component, by means respectively of a transition fit and an interference fit. The inner surface of the side wall on which the protrusions are provided may diverges from the front end towards the opening of the cavity. The cavity may have a first width at the first protrusion and a second width at the second protrusion, the first width being smaller than the second width.

The applicator tip may be formed as a single piece including the rear end, the front end and the side wall, the front end comprising a hole for receiving the cannula.

Alternatively, the applicator tip may be formed as a single piece including the rear end, the front end, the side wall and the cannula.

According to embodiments of the invention, the applicator tip is configured so that the side wall is deflectable in a direction transversal to its longitudinal axis, i.e., an axis extending from the front end to the rear end. Advantageously, this facilitates the coupling with the storing component.

According to a third aspect of the invention, a storing component for an applicator, in particular for the applicator above described, is provided. The storing component comprises a container for storing a fluid material, a front portion configured to be housed in a cavity of an applicator tip of the applicator. The front portion comprises at least one connection element for providing a removable connection between the front portion and the cavity of the applicator tip, said removable connection comprising at least an interference fit or a snap-fit and a fluid-tight element for providing a fluid-tight connection between a cannula of the applicator tip and the storing component for fluidically coupling the cannula to the container.

The storing component may include any of the features above described with reference to the storing component of the applicator according to the first aspect of the invention.

In particular, the front portion comprises a first mating surface and a second mating surface, the first mating surface and the second mating surface being configured and dimensioned for providing a removable connection by coupling with a respective first protrusion and a second protrusion provided in the cavity of the applicator tip. The first mating surface is configured and dimensioned for coupling to the first protrusion by means of a transition fit and the second mating surface is configured and dimensioned are for coupling to the second protrusion by means of an interference fit.

According to embodiments of the storing component, the fluid-tight element of the storing component may be the valve including a membrane, as above described with reference to the applicator. According to some embodiments, the fluid-tight element of the storing component may be the inner duct receiving the front end of the cannula. According to some embodiments, the fluid-tight element of the storing component may be the orifice directly coupling with a first axial end of the cannula.

According to embodiments of the storing component, the storing component further comprises an applicator closure tip including a cavity defining a housing for the front portion, a rear end comprising an opening for allowing the front portion to be inserted into the cavity, a front end comprising a closed front wall defining a bottom of the cavity, a side wall extending between the rear end and the front end. The storing component is configurable in at least a first configuration and a second configuration. In the first configuration the front portion is outside the cavity and the front portion is attached to the applicator closure tip by means of a breakable connection for providing a closure to the orifice. In the second configuration the front portion is housed in the cavity of the applicator closure tip, at least one removable connection being provided between a first connection member on the front portion and a second connection member on the side wall of the closure tip, a fluid - tight contact being provided between the closed front wall of the closure tip and the front portion for closing the orifice.

Advantageously, the storing component can be manufactured with the applicator closure tip in the first configuration. The container is then filled with the fluid material. Upon first use of the applicator, the breakable connection is broken and the applicator closure tip is removed from the storing component, thus opening the storing component. Afterwards an applicator tip, as above described, may be connected to the front portion of the storing component. After the dispensing of the fluid material, in particular if the container is not empty, the applicator tip may be removed and the storing component by means of the applicator closure tip in the second configuration.

According to embodiments of the storing component, the closed front wall of the cavity of the applicator closure tip comprises a front protrusion for closing the orifice in the second configuration. The front protrusion may be conically shaped, for example, the front protrusion may be provided as a truncated cone.

In the following, exemplary embodiments of the present disclosure are described in conjunction with the drawings, showing schematically:
- Fig. 1: a perspective view of an applicator according to one embodiment of the present invention, including an applicator tip and a storing component;
- Fig. 2: a cross-sectional side view of the applicator of Fig. 1 in one operative configuration;
- Fig. 3: a perspective view of a storing component according to one embodiment of the present invention;
- Fig. 4: a perspective view of a storing component according to another embodiment of the present invention;
- Fig. 5: a perspective view of an applicator tip according to one embodiment of the present invention;
- Fig. 6: a first cross-sectional side view of the applicator tip of Fig. 5;
- Fig. 7: a second cross-sectional side view of the applicator tip of Fig. 5;
- Fig. 8: a perspective view of a component of the storing component;
- Fig. 9: a perspective of another embodiment of the component of Fig. 5;
- Fig. 10: a cross-sectional side view of the storing component;
- Fig. 11: a cross-sectional side view of the applicator of Fig. 1 in another operative configuration;
- Fig. 12: a perspective view of an applicator according to another embodiment of the present invention;
- Fig. 13: a cross-sectional side view of the applicator of Fig. 9 in one operative configuration;
- Fig. 14: a magnified view of the detail XI of Fig. 10;
- Fig. 15: a cross-sectional side view of the applicator of Fig. 10 in another operative configuration;
- Fig. 16: a cross-sectional side view of an applicator according to a further embodiment of the present invention;
- Fig. 17: a side view of a storing component according to another embodiment of the present invention;
- Fig. 18: a cross-sectional side view of the storing component of fig. 14, in a first configuration;
- Fig. 19: a cross-sectional side view of the storing component of fig. 14, in a second configuration;
- Fig. 20: a cross-sectional side view of the storing component of fig. 14, in a third configuration.

In the following, an applicator 10 and advantageous aspects thereof are described with reference to the accompanying drawings.

The applicator 10 is usable to house and dispense a fluid material to be used in medical and dental applications. In particular, the material dispensable by the applicator 10 may be for example at least one of 1K materials, fluids, cremes, medical fluids, etching materials, adhesives, ointments, paints and the like. The applicator 10 can be used a number of times and can be referred to as a multi-use applicator.

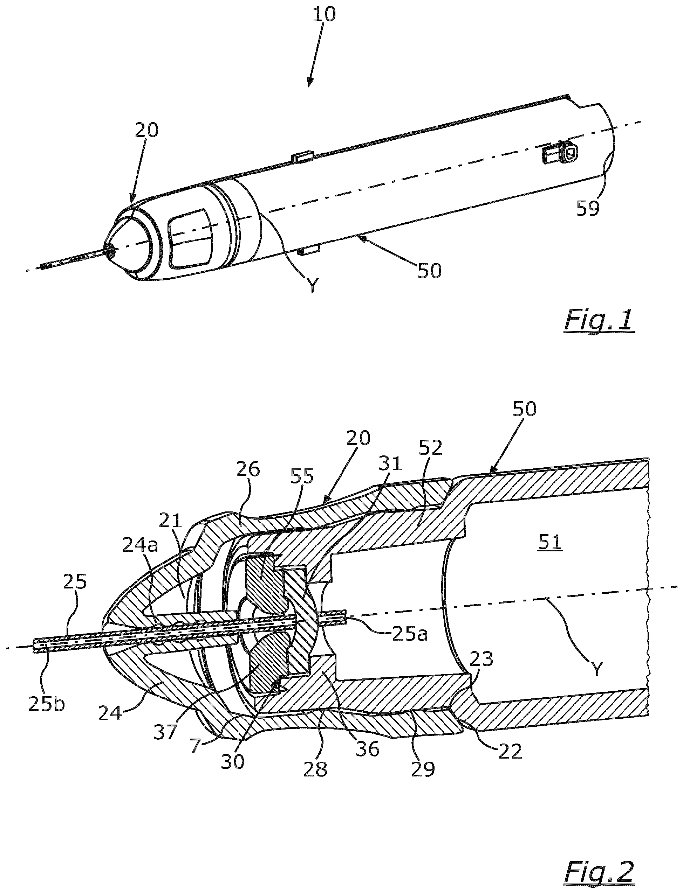

As shown in Fig. 1, an applicator 10 includes an applicator tip 20 and a storing component 50. The applicator tip 20 is removably attachable to the storing component 50 by means of a removable connection comprising at least an interference fit or a snap-fit, as further described in the following. The applicator 10 longitudinally extends along a longitudinal axis Y.

As shown in Fig. 2, the applicator tip 20 comprises a cavity 21 defining a housing for a front portion 52 of the storing component 50. The front portion 52 of the storing component 50 is housed in the cavity 21, when the applicator tip 20 is attached to the storing component 50 by means of the removable connection. The applicator tip 20 further comprises a rear end 22, comprising an opening 23 for allowing the front portion 52 of the storing component 50 to be inserted into the cavity, and a front end 24 defining a bottom of the cavity 21. The applicator tip 20 further comprises a side wall 26, extending between the rear end 22 and the front end 24 and a cannula 25 extending through the front end 24 between a first cannula end 25a and a second cannula end 25b.

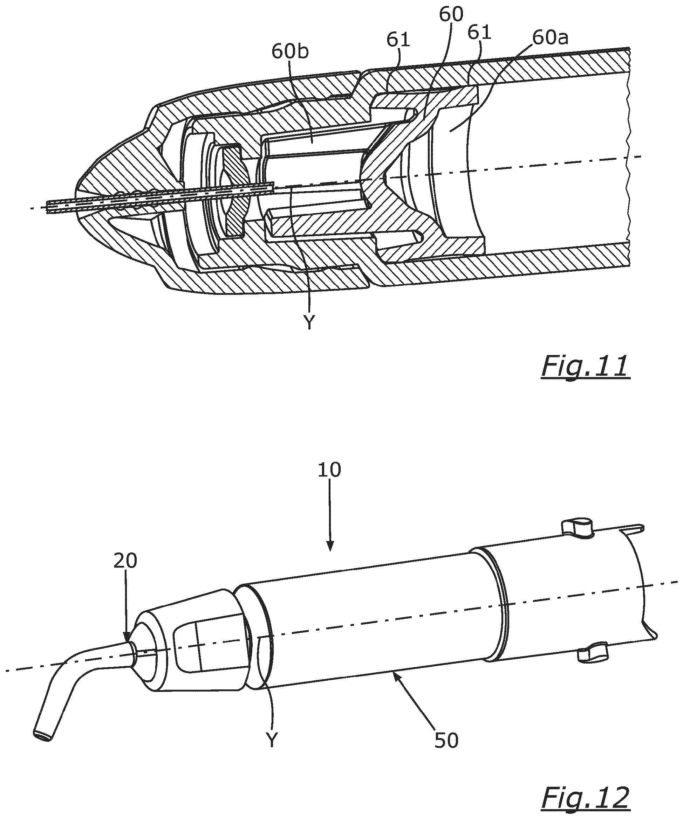

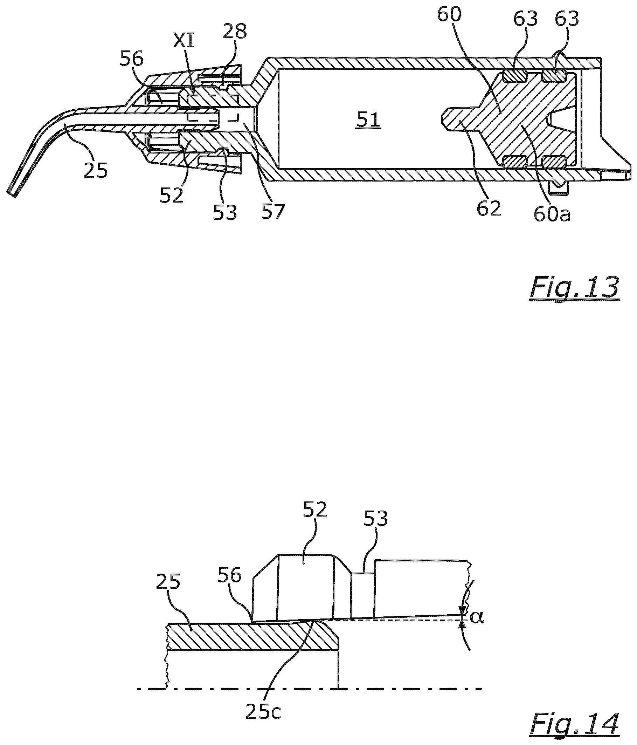

The first cannula end 25a of the cannula 25 is located in the cavity 21. The second end 25b of the cannula 25 protrudes from the front end 24, outside the cavity 21. The cannula 25 is linear and aligned with the longitudinal axis Y when the applicator tip 20 is attached to the storing component 50. According to other embodiments (for example, as shown in Figs. 12 to 16) of the invention, the cannula 25 is curved or comprises two portions inclined with respect to each other.

The rear end 22, the front end 24 and the side wall 26 of the applicator tip 20 are formed as a single piece, for example as a single piece of plastic material, which may be obtained by injection molding. The front end 24 comprises a hole 24a for receiving the cannula 25. The cannula 25 is fixedly coupled to the hole 24a, for example by means of an interference fit. According to other embodiments (for example, as shown in Figs. 12 to 16) of the invention, the rear end 22, the front end 24, the side wall 26 and the cannula 25 of the applicator tip 20 are formed as a single piece, for example as a single piece of plastic material, which may be obtained by injection molding.

With reference to Figs. 1 to 3, the storing component 50 comprises a container 51 for storing the fluid material to be dispensed through the cannula 25 of the applicator tip 20. The storing component 50 further includes the front portion 52 configured to be housed in the cavity 21 of the applicator tip 20 and a longitudinally opposed rear portion 59.

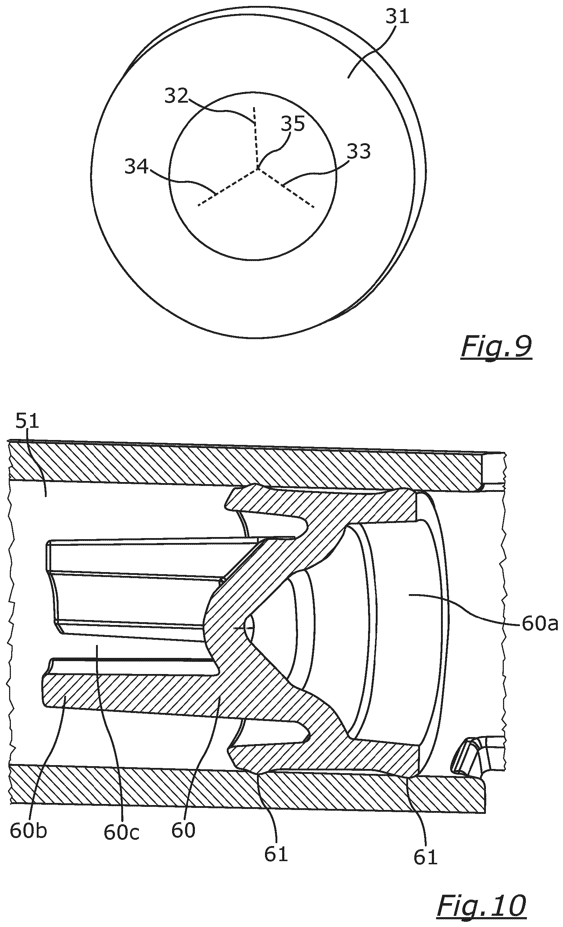

Inside the container 51, a piston 60 (one embodiment of the piston is shown in Figs. 10 and 11) is movable from the rear portion 59 to front portion 52 to push the fluid material to be delivered towards front portion 52. The piston is operated by means of a dispenser removably attachable to the rear portion 59 of the storing component 50. The dispenser and the coupling between dispenser and storing component 50 are not a specific object of the present invention and therefore are not described in more detail.

For providing the removable connection between the applicator tip 20 and the storing component 50, a first protrusion 28 and a second protrusion 29 are provided on the side wall 26 of the applicator tip 20, and a first mating surface 53 and a second mating surface 54 are provided on the front portion 52 of the storing component 50.

Alternatively, according to other embodiments (not shown in the attached figures), the first protrusion 28 and the second protrusion 29 are provided on the front portion 52 of the storing component 50, and the first mating surface 53 and the second mating surface 54 are provided on the side wall 26 of the applicator tip 20.

The first protrusion 28 and the first mating surface 53 are configured and dimensioned for coupling to each other when the front portion 52 of the storing component is housed in the cavity 21 and the second protrusion 29 and the second mating surface 54 are configured and dimensioned for coupling to each other when the front portion of the storing component is housed in the cavity 21.

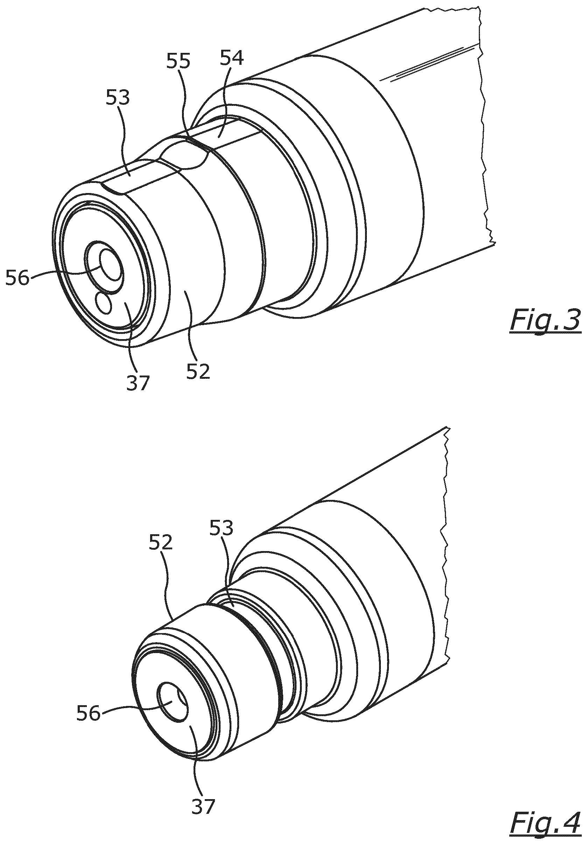

With reference to the embodiment of Figs. 2 and 3, the side wall 26 comprises an inner surface 27 delimiting the cavity 21 and the side wall 26 comprises the first protrusion 28 protruding in the cavity 21 from the inner surface 27 and the second protrusion 29 protruding in the cavity 21 from the inner surface 27. The first protrusion 28 is closer to the front end 24 than the second protrusion 29. The inner surface 27 the side wall 26 diverges from the front end 24towards the opening 23 of the cavity 31. The cavity 31 has a first width at the first protrusion 28 and a second width at the second protrusion 29, the first width being smaller than the second width.

The front portion 52 of the storing component 50 comprises the first mating surface 53 and the second mating surface 54, positioned with respect to each other, so that the second mating surface 54 is closer to the rear portion 59 of the storing component 50 than the first mating surface 53. The first protrusion 28 and the first mating surface 53 are configured and dimensioned for coupling to each other by means of a transition fit and the second protrusion 29 and the second mating surface 54 are configured and dimensioned are configured and dimensioned for coupling to each other by means of an interference fit.

The applicator tip 20 extends from the front end 24 to the rear end 22 along a longitudinal axis, which is aligned or parallel to the longitudinal axis Y when the applicator tip 20 is coupled to the storing component 50. The side wall 26 is deflectable in a direction transversal to the longitudinal axis Y. This facilitates the coupling of the first protrusion 28 with the first mating surface 53 and of the second protrusion 29 with the second mating surface 54.

According to embodiment of the present invention, the applicator tip 20 is formed as a single piece, for example by means of injection moulding. The material the applicator tip 20 is chosen to facilitate the deflection in the direction transversal to the longitudinal axis Y.

The second mating surface 54 comprises an edge 55 protruding from the second mating surface 54 and interposed between the container 51 and the first mating surface 53. The edge 55 is also interposed between the second protrusion 29and the first protrusion 28 when the front portion 52 of the storing component 50 is housed in the cavity 21, with the first protrusion 28 and the first mating surface 53 coupled to each other and the second protrusion 29 and the second mating surface 54 coupled to each other. This arrangement provides a snap-fit between the edge 55 and the second protrusion 29. The interference between the second projection 29 and the edge 55 provides an obstacle to an inadvertent removal of the coupling between the applicator tip 20 and the storage component 50. According to other embodiments of the storing component 50, the edge 55 is not present, the coupling between the applicator tip 20 and the storage component 50 is only performed through by means of the first protrusion 28 and the second protrusion 29 and the first mating surface 53 and the second mating surface 54, as described above.

With reference to Fig. 3, the first mating surface 53 and the second mating surface 54 are provided as respective depressions on respective external cylindrical or conical surfaces of the front portion 52 of the storing component 50. According to embodiments of the present invention, the first mating surface 53 and the second mating surface 54 are provided as channels or grooves or concave recesses or flattened portion along the front portion 52 of the storing component 50. The first mating surface 53 and the second mating surface 54 are longitudinally oriented, i.e., parallel or substantially parallel to the longitudinal axis Y, so that the first protrusion 28 and the second protrusion 29 are guided when the applicator tip 20 is coupled to the front portion 52. This improves the handling of the applicator.

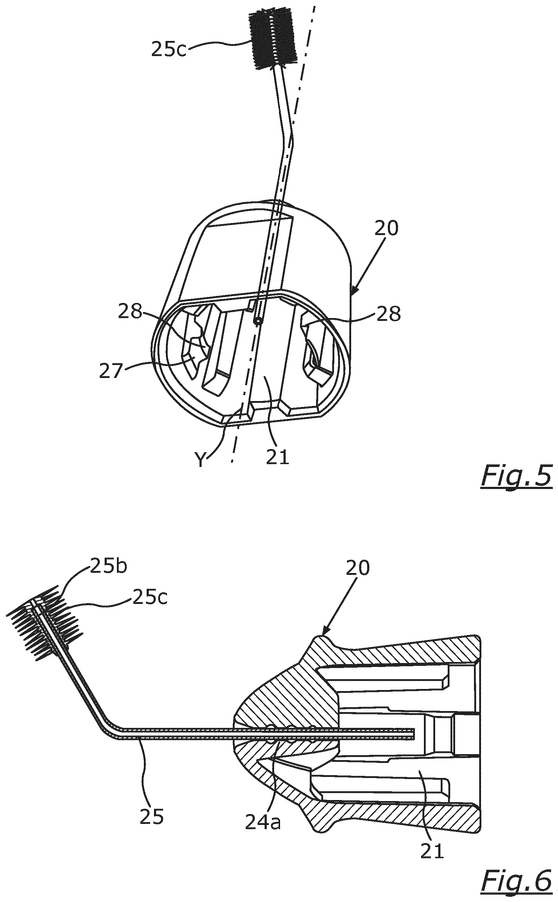

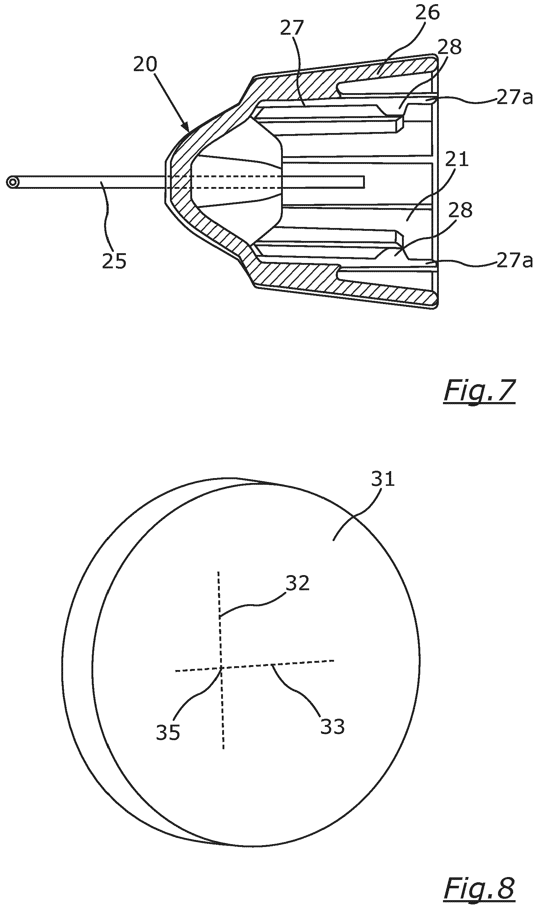

With reference to Fig. 4, according to another embodiment of the storing component 50, one mating surface 53 is provided, to be coupled to one or more respective protrusions 28 (Fig. 5 and 7) protruding in the cavity 21 from the inner surface 27 of the applicator tip 20. In such embodiment, the mating surface 53 is provided as a circumferential recess or groove, i.e., a recess or groove that is orthogonal or substantially orthogonal to the longitudinal axis Y. As shown in Fig. 4, the mating surface 53 is provided as an annular circumferential groove extending for 360° about the longitudinal axis Y. According to other embodiments (not shown), the circumferential groove may extend for an angle of less than 360° about the longitudinal axis Y. According to another embodiment (not shown) two mating surfaces are provided as a circumferential recesses or grooves. Mating surfaces configured as circumferential recess or groove are used for providing snap-fir connections between the cavity 21 and the front portion 52.

Similar embodiments of the invention are shown in Figs. 12 to 16, where the mating surface 53 is provided as a circumferential recess or groove and the removable connection between the cavity 21 and the front portion 52 is provided by means of a snap-fit connection.

With reference to Figs. 5 to 7, another embodiment of the applicator tip 20 is shown, which is particularly, but not exclusively, suitable for being coupled with the storing component 50 of Fig. 4. According to such embodiments the cannula 25 comprises a brush 25c, or other end tool, attached to the second cannula end 25b. The brush is usable 25c to clean a surface on which the fluid material is to be subsequently dispensed through the cannula 25. With respect to the longitudinal axis Y, two diametrically opposed protrusions 28 are provided in the cavity 21 for coupling with the mating surface 53. Each protrusion 28 extends orthogonally to the longitudinal axis Y from a respective leg 27a longitudinally protruding in the cavity 21 from the side wall 26. The deflection of the legs 27a orthogonally to the longitudinal axis Y facilitates the coupling between the protrusion 28 and the mating surface 53. According to another embodiment (not shown) more than two protrusion 28 may be provided. According to another embodiment (not shown) only one protrusion may be provided, for example, extending for 360° about the longitudinal axis Y.

A fluid-tight connection is provided between the cannula 25 and the storing component 50 for fluidically coupling the cannula 25 to the container 51, when the applicator tip 20 and the storage component 50 are coupled to each other.

With reference to the embodiments of Figs. 2 to 4, the front portion 52 comprises a valve 30 fluid-tightly coupled with the first end 25a of the cannula 25 of the applicator tip, when the front portion 52 of the storing component 50 is housed in the cavity 21. The valve 30 comprises a membrane 31 which is made in a material impermeable to the fluid material inside the container 51. Embodiments of the membrane are shown in Figs. 8 and 9. The membrane 31 comprises an elastic opening 32 for allowing the first end 25a of the cannula 25 to be inserted in the container 52, when the front portion 52 of the storing component 50 is housed in the applicator tip 20. The membrane 31 may be flat are convex. In particular, as shown in the figures, the membrane 31 is convex towards an inside of the container 51, i.e., the membrane 31 comprises a concave surface on the opposite side facing the cavity 21 when the applicator tip 20 is attached to the storing component 50.

The membrane 31 is shaped as a disc which is fixed to the front portion 52 of the storing component 50 along the peripheral edge of the membrane 31. The Alternatively, according to other embodiments (not shown) the membrane 31 may may have a different shape. The peripheral edge of the membrane 31 is fixed between a first and a second ring 36, 37 radially protruding, with respect to the longitudinal axis Y, from an inner surface of the front portion 52. The axial space between the first and a second ring 36, 37 provides a seat for the membrane 31. The first ring 36 is more internal, i.e., closer to the rear portion 59 of the storing component 50 than the second ring 37, and is provided as an integral portion of the front portion 52. The second ring 37 is provided as a closure element which is fixed to the front portion 52 so that the membrane is longitudinally blocked between the first and the second ring 36, 37. The central hollows portions of the first and the second ring 36, 37 provides a passage, which permits inserting the cannula 25 to pass through valve 30 and to be inserted in the container 52. The central hollow portion of the second ring 37 provides a frontal orifice 56 of the storing component 50 for the insertion of the cannula 25 in the storing component 50. According the other embodiments (shown in Figures 8 to 15), the second ring 37 is not present and the orifice 56 is provided as an opening of the front portion 52 of the storing component 50.

In the embodiment of Fig. 8, the elastic opening of the membrane 31 comprises two cuts 32, 33 crossing each other at a point of intersection 35. The two cuts 32, 33 are angularly offset with respect to one another about the longitudinal axis Y of an angle of 90°, i.e., the two cuts 32, 33 are perpendicular to each other in a plane perpendicular to the longitudinal axis Y. According to other embodiments (not shown) the two cuts 32, 33 are angularly offset with respect to one another about the longitudinal axis Y of an angle greater or smaller than 90°.

In the embodiment of Fig. 9, the elastic opening of the membrane 31 comprises three cuts 32, 33, 34 crossing each other at a point of intersection 35. The three cuts 32, 33, 35 are angularly offset with respect to one another about the longitudinal axis Y of an angle of 120°. According to other embodiments (not shown), the elastic opening 32 may provided according to other shapes and dimensions with permits a fluid tight-coupling with the cannula 25.

When the applicator tip 20 is attached to the storing component 50, the cannula 25 is forced towards the membrana 31 and the opening 32. The opening 32 permits the passage of the cannula 25 through the membrane 31. The elasticity of the material of the membrane 31 allows the edges of the opening to adhere to the membrane 31, so that a fluid-tight coupling is provided. When the cannula 25 is coupled to the membrane 31, the impermeability of the membrane 31 to the fluid material inside the container 51, allows the fluid material in the container 31 to be directed through the cannula 25 for dispensing.

The removable connections between the cavity 21 and the front portion 52 of the storing component 50 are configured for allowing a smooth coupling between the applicator tip 20 and the the storing component 50, so that pressure equalization in the applicator tip 20 is provided during attachment to or removal from the front portion 52. This avoids rapid pressure fluctuations, which could cause the valve 30 to open. This is achieved, for example, by conveniently dimensioning the first mating surface 53 and the second mating surface 54 as longitudinal channels or grooves or concave recesses or flattened portion (Fig. 3). The elasticity properties of the applicator tip 20 may further enhance pressure equalization.

With reference to Figs. 12 to 15, another embodiment of the fluid-tight coupling between the cannula 25 and the front portion 52 of the storing component 50 is described. In such embodiment, the fluid-tight connection being provided between the cannula 25 and an inner duct 57 of the front portion 52, the inner duct 57 being provided in the front portion 52 for connecting the container 51 to the orifice 56 of the front portion 52. The inner duct 57 extends along a longitudinal axis of the inner duct 57, which may be coincident or parallel to the longitudinal axis Y of the applicator 10. A portion of the cannula 25 including the first end 25a is inserted in the inner duct 57 of the front portion 52, when the applicator tip 20 is attached to the storing component 50. A protrusion 25c radially protruding from the first end 25a of the cannula 25 contacts with the inner duct 57 to provide the fluid - tight connection between the cannula 25 and the storing component 50. The inner duct 57 of the front portion 52 is divergent from the orifice 56 to the container 51. The inclination of the inner duct 57 is inclined of an angle α comprised between 1° and 3° with respect to the longitudinal axis Y. The pressure of the fluid material inside the container 51 acts against the first end 25a of the cannula 25, which is forced back from the container 51 and towards the orifice 56. The inclination of the inner duct 57, which is convergent from the container 51 to the orifice 56, counteracts the fluid pressure. As a result, the fluid tightness of the contact between the cannula 25 and the inner duct 57 is increased. The first cannula end 25a may comprise an external chamfer 25d for facilitating the insertion of the first end 25a of the cannula 25 into the inner duct 57.

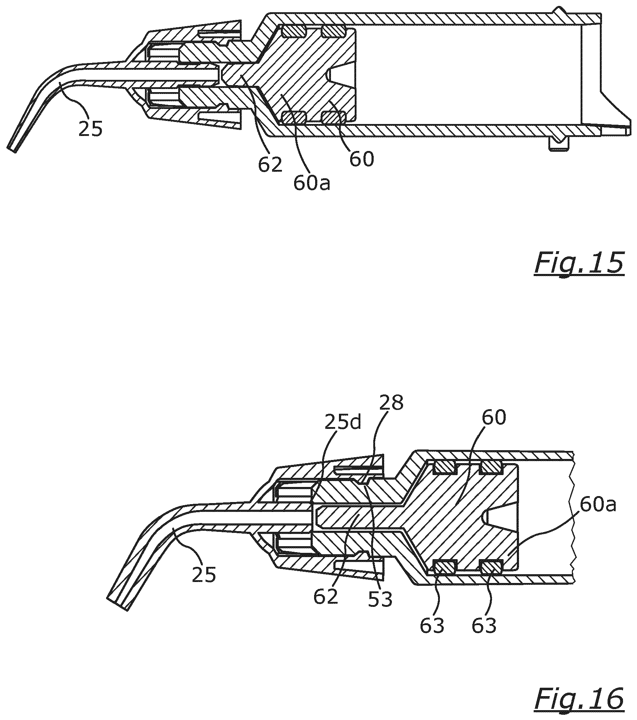

With reference to Fig. 16, another embodiment of the fluid-tight coupling between the cannula 25 and the front portion 52 of the storing component 50 is described. Such embodiment differentiates itself from the previously described embodiment, in that the first cannula end 25a comprises an external chamfer 25d, which is dimensioned to contact the orifice 56 to provide said fluid - tight coupling between the cannula 25 and storing component 50. In other words, the dimension of the first end 25a of the cannula 25 allows only a portion of the chamfered first end 28a of the cannula 25 to be inserted in the inner duct 57, thus providing the fluid -tight coupling with the inner duct 57. The external chamfer of the first cannula end 25a is inclined of an angle comprised between 20° and 60° with respect to the axis of the inner duct 57.

With reference to Figs. 10 and 11, an embodiment of the piston 60, which is movable in the container 51 for dispensing the fluid material, is described. The piston 60 comprises a main body 60a and an axial protrusion 60b, axially extending main body 60a towards the front portion 52 of the storing component 50.

The main body 60a longitudinally extends between two axial ends, where two respective sealing lips 61 are provided as radial protrusions for providing a seal between the piston 60 and the container 51. The sealing lips 61 are formed in one piece with the piston 60, for example by injection moulding. The piston 60 axially moves inside the container 51 between a first rear position close to the rear portion 59 of the storing component 50 to a second front position close to the valve 30, in which the axial protrusion 60b is inserted in the front portion 52 of the storing component 50 and abuts the first ring 36 of the storing component 50.

The axial protrusion 60b is hollow, so that the cannula 25 does not interfere with the piston 60, when the piston 60 reaches the second front position. The first rear position of the piston 60 corresponds to a full state of the container 51, before the dispensing of the fluid material, while the second front position of the piston 60 corresponds to an empty state of the container 51, which is reached at the end of the dispensing of the fluid material.

The axial protrusion 60b comprises recesses 60c extending from one of the axial ends towards the main body 60a. By way of example two to six such recesses 60c can be provided.

The recesses 60c are provided in order to facilitate a dispensing of fluid material via the cannula 25, as they impart a flexibility to the axial protrusion 60b.

The axial protrusion 60b has an at least generally truncated cone shaped outer shape, whereas the main body 60a has a generally cylindrical outer shape.

The axial protrusion 60b is shaped such that is fits snuggly into the front portion 52 of the storing component 50, whereas the main body 60a is shaped to move within the container 51 such that the main body 60a forms a seal between the container 51 and the piston 60. Thereby the lips 61 avoid fluid material from exiting the piston 60 via one axial end.

With reference to Figs. 12 to 16, another embodiment of the piston 60 is described. In such embodiment, the piston 60 longitudinally extends between two axial ends, each axial end being provided with a respective gasket 63, for example a respective O-ring, for providing a seal between the piston 60 and the container 51.

The gaskets 63 may be mounted on respective seats provided on the main body 60a of the piston 60 or be formed in one piece with the piston 60, for example gaskets 63 may be overmoulded to the piston 60. The piston 60 further comprises a front end having a piston axial protrusion 62 at least partially insertable in the inner duct 57. The piston 60 axially moves inside the container 51 between a first rear position close to the rear portion 59 of the storing component 50 to a second front position close to the cannula 25, in which the axial protrusion 63 is inserted in the inner duct 57 and is adjacent to the first end 25a of the cannula 25. Analogously to the embodiment of figs. 10 and 11, the first rear position of the piston 60 corresponds to a full state of the container 51, before the dispensing of the fluid material, while the second front position of the piston 60 corresponds to an empty state of the container 51, which is reached at the end of the dispensing of the fluid material.

The axial protrusion 62 is shaped such that is fits snuggly into the inner duct 57 of the storing component 50, whereas the main body 60a is shaped to move within the container 51 such that the main body 60a forms a seal between the container 51 and the piston 60. Thereby the gaskets 63 avoid fluid material from exiting the piston 60 via one axial end.

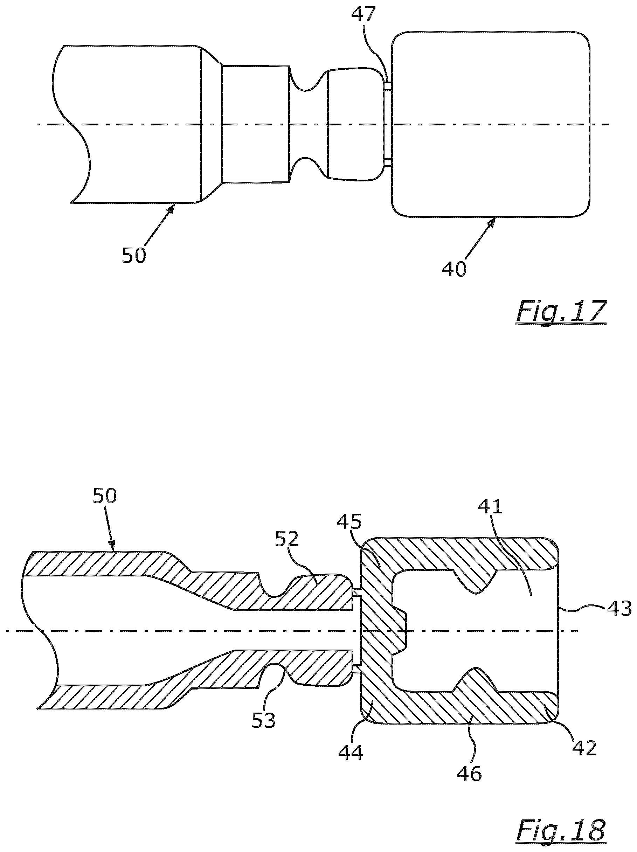

With reference to Figs. 17 to 20, another embodiment of the storing component 50 is described. The storing component 50 further comprises an applicator closure tip 40 for providing a closure to the orifice 56. The applicator closure tip 40 comprises a cavity 41 defining a housing for the front portion 52, a rear end 42 comprising an opening 43 for allowing the front portion 52 to be inserted into the cavity 41 of the applicator closure tip 40, a front end 44 comprising a closed front wall 45 defining a bottom of the cavity 41 and a side wall 46 extending between the rear end 42 and the front end 44. The storing component 50 according to this embodiment is configurable in at least a first configuration and a second configuration. In the first configuration the front portion 52 is outside the cavity 41 of and the front portion 52 is attached to the applicator closure tip 40 by means of a breakable connection 47, which provides a closure to the orifice 56. The assembly of the storing component 50 with the applicator closure tip 40 in the first configuration is formed in one piece, for example by injection moulding. In the second configuration the front portion 52 is housed in the cavity 41 of the applicator closure tip 40. A removable connection is provided between a first connection member 53, for example a mating surface configured as a circumferential recess or groove, on the front portion 52 and a second connection member 48, for example a protrusion, on the side wall 46 of the applicator closure tip 40. Alternatively, the first connection member 53 may be a protrusion and the second connection member 48 may be a recess or groove. The removable connection may be an interference fit or a snap-fit. More than one removable connection may be provided between the front portion 52 and the applicator closure tip 40. A fluid - tight contact is provided in the second configuration between the closed front wall 45 of the applicator closure tip 40 and the front portion 52 for closing the orifice 56. The closed front wall 45 of the cavity 41 comprises a front protrusion 49 for closing the orifice 56 in the second configuration. The front protrusion 49 is shaped as a cone or a truncated cone, which provides the fluid -tight coupling with the orifice 56. The cone or truncated cone is inclined of an angle comprised between 20° and 60° with respect to the axis of the longitudinal axis Y.

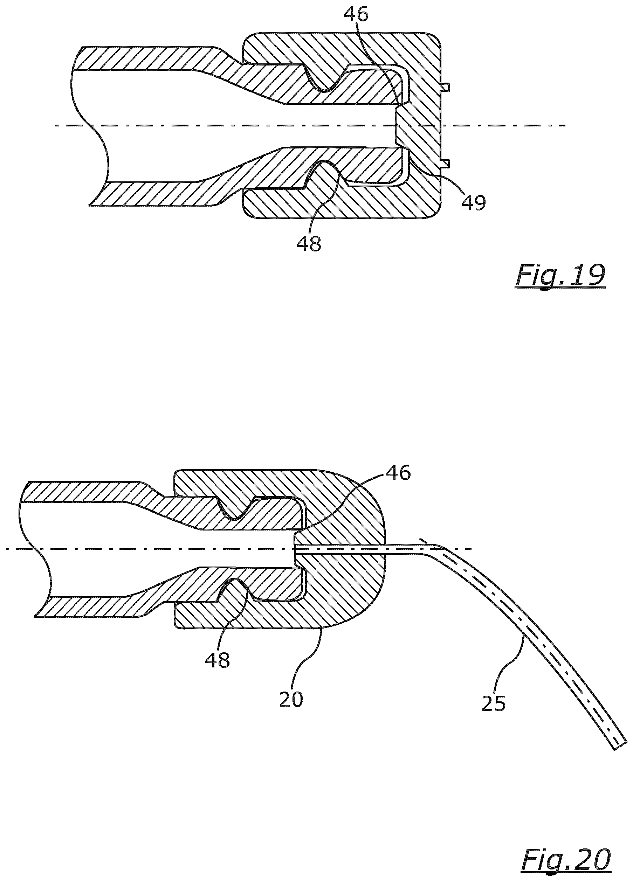

For an applicator 10 including the storing component 50 of Figs. 17 to 20, the storing component 50 is manufactured with the applicator closure tip 40 in the first configuration (Fig. 18). The container 51 is filled with the fluid material, for example from the rear portion 59 and the piston 60 in inserted in the container 51. Upon first use of the applicator, the breakable connection 47 is broken and the applicator closure tip 40 is removed from the storing component 50, thus opening the storing component 50. Afterwards an applicator tip 20, as above described, may be connected to the front portion 52 of the storing component 50, thus reaching a third configuration (Fig. 20). For connecting the applicator tip 20 to the storing component 50 the first connection member 53 on the front portion 52 of the storing component 50 may be used, as above described with reference to the embodiments of Figs. 1 to 16. After the dispensing of the fluid material, in particular if the container 51 is not empty, the applicator tip 20 may be removed and the storing component 50 by means of the applicator closure tip 40 in the second configuration (Fig. 19).

### Enumerated embodiments:

1. An applicator (10) including an applicator tip (20) and a storing component (50), the applicator tip (20) comprising:
   a cavity (21) defining a housing for the storing component (50),
   a rear end (22) comprising an opening (23) for allowing the storing component (50) to be partially inserted into the cavity (21),
   a front end (24) defining a bottom of the cavity (21),
   a side wall (26) extending between the rear end (22) and the front end (24), and
   a cannula (25) extending through the front end (24) between a first cannula end (25a) and a second cannula end (25b), the first cannula end (25a) being located in the cavity (21);
   the storing component (50) comprising:
   a container (51) for storing a fluid material and
   a front portion (52) configured to be housed in the cavity (21) of the applicator tip (20),
   wherein
   a removable connection is provided between the cavity (21) and the front portion (52) of the storing component (50), the removable connection comprising at least an interference fit or a snap-fit, and
   a fluid-tight connection is provided between the cannula (25) and the storing component (50) for fluidically coupling the cannula (25) to the container (51).
2. The applicator (10) according to embodiment 1, wherein the removable connection comprises at least a first protrusion (28, 29) on one of the side wall (26) of the applicator tip (20) and the front portion (52) of the storing component (50), and the removable connection comprises at least a first mating surface (53, 54) on the other of the side wall (26) of the applicator tip (20) and the front portion (52) of the storing component (50), and wherein the first protrusion (28, 29) and the first mating surface (53, 54) are configured and dimensioned for coupling to each other when the front portion (52) of the storing component is housed in the cavity (21).
3. The applicator (10) according to embodiment 2, wherein the removable connection comprises the first protrusion (28) and at least a second protrusion (29) on the side wall (26) and the removable connection comprises the first mating surface (53) and at least a second mating surface (54) on the front portion (52) of the storing component (50), the second protrusion (29) and the second mating surface (54) being configured and dimensioned for coupling to each other when the front portion (52) of the storing component is housed in the cavity (21), and
   wherein the first protrusion (28) is closer to the front end (24) than the second protrusion (29).
4. The applicator (10) according to embodiment 3, wherein the first protrusion (28) and the first mating surface (53) are configured and dimensioned for coupling to each other by means of a transition fit and the second protrusion (29) and the second mating surface (54) are configured and dimensioned are configured and dimensioned for coupling to each other by means of an interference fit.
5. The applicator (10) according to embodiment 4, wherein the second mating surface (54) comprises an edge (55) protruding from the second mating surface and interposed between the second protrusion (29) and the first protrusion (28) when the front portion (52) of the storing component is housed in the cavity (21) with the first protrusion (28) and the second protrusion (29) respectively coupled to the first mating surface (53) and the second mating surface (54).
6. The applicator (10) according to any of the previous embodiments, wherein the front portion (52) comprises a valve (30) fluid-tightly coupled with the first end (25a) of the cannula (25) of the applicator tip when the front portion (52) of the storing component is housed in the cavity (21).
7. The applicator (10) according to embodiment 6, wherein the valve (30) comprises a membrane (31) which is impermeable to the fluid material inside the container (51), the membrane (31) comprising an elastic opening (32) for allowing the first end (25a) of the cannula (25) to be inserted in the container (51) when the front portion (52) of the storing component (50) is housed in the applicator tip (20).
8. The applicator (10) according to embodiment 1, wherein the front portion (52) includes an orifice (56) and an inner duct (57) for connecting the container (51) to the orifice (56), a fluid-tight connection being provided between the cannula (25) and the inner duct (57).
9. The applicator (10) according to embodiment 8, wherein the first cannula end (25a) comprising an external chamfer contacting the orifice (56) to provide said fluid - tight coupling.
10. The applicator (10) according to embodiment 9, wherein the external chamfer of the first cannula end (25a) is inclined of an angle comprised between 20° and 60° with respect to an axis of the cannula (25).
11. The applicator (10) according to embodiment 8 or 9, wherein the cannula (25) is partially inserted in the inner duct (57) of the front portion (52), the cannula (25) contacting with the inner duct (57) to provide said fluid - tight connection.
12. The applicator (10) according to embodiment 11, wherein the inner duct (57) of the front portion (52) is divergent from the orifice (56) to the container (51).
13. The applicator (10) according to embodiment 12, wherein the inner duct (57) is inclined of an angle comprised between 1° and 3° with respect to an axis of the inner duct (57).
14. An applicator tip (20) for an applicator (10), the applicator tip (10) comprising:
   a cavity (21) defining a housing for the storing component (50) of the applicator (10),
   a rear end (22) comprising an opening (23) for allowing the storing component (50) to be partially inserted into the cavity (21),
   a front end (24) defining a bottom of the cavity (21),
   a side wall (26) extending between the rear end (22) and the front end (24), the side wall (26) comprising an inner surface delimiting the cavity (21), and
   a cannula (25) extending through the front end (24) between a first cannula end (25a) and a second cannula end (25b), the first cannula end (25a) being located in the cavity (21);
   wherein,
   the side wall (26) comprises at least one connection element (28, 29) for providing a removable connection between the cavity (21) and the front portion (52) of the storing component (50), said removable connection comprising at least an interference fit or a snap-fit.
15. The applicator tip (20) according to embodiment 14, wherein the side wall (26) comprises an inner surface (27) delimiting the cavity (21) and the side wall (26) comprises a first protrusion (28) protruding in the cavity (21) from the inner surface (27) and a second protrusion (29) protruding in the cavity (21) from the inner surface (27), the first protrusion (28) being closer to the front end (24) than to the second protrusion (29), the first protrusion (28) and the second protrusion (29) being configured and dimensioned for providing said removable connection by coupling with a respective first mating surface (53) and a second mating surface (54) provided on the front portion (52) of the storing component (50).
16. The applicator tip (20) according to embodiment 15, wherein the first protrusion (28) is configured and dimensioned for coupling to the first mating surface (53) by means of a transition fit
17. The applicator tip (20) according to embodiment 15 or 16, wherein the second protrusion (29) is configured and dimensioned are for coupling to the second mating surface (54) by means of an interference fit.
18. The applicator tip (20) according to embodiment 15 or 16 or 17, wherein the inner surface (27) of the side wall (26) diverges from the front end (24) towards the opening (23) of the cavity (31).
19. The applicator tip (20) according to any of the embodiments 14 to 18, wherein the cavity (31) has a first width at the first protrusion (28) and a second width at the second protrusion (29), the first width being smaller than the second width.
20. The applicator tip (20) according to any of the embodiments 14 to 19, wherein the applicator tip (20) extends from the front end (24) to the rear end (22) along a longitudinal axis (Y) and the side wall (26) is deflectable in a direction transversal to the longitudinal axis.
21. The applicator tip (20) according to any of the embodiments 14 to 20, wherein the applicator tip (20) is formed as a single piece.
22. The applicator tip (20) according to any of the embodiments 14 to 21, wherein the rear end (22), the front end (24) and the side wall (26) are formed as a single piece, the front end (24) comprising a hole (24a) for receiving the cannula (25).
23. A storing component (50) for an applicator (10), the storing component comprising:
   a container (51) for storing a fluid material, and a front portion (52) configured to be housed in a cavity (21) of an applicator tip (20) of the applicator (10),
   wherein the front portion (52) comprises
      at least one connection element (53, 54) for providing a removable connection between the front portion (52) and the cavity (21) of the applicator tip (20), said removable connection comprising at least an interference fit or a snap-fit, and
      a fluid-tight element (30, 57) for providing a fluid-tight connection between a cannula (25) of the applicator tip (20) and the storing component (50) for fluidically coupling the cannula (25) to the container (51).
24. The storing component (50) according to embodiment 23, wherein the storing component (50) extends from the front portion (52) to a rear portion (59) along a longitudinal axis (Y), and wherein the at least one connection element (53, 54) is configured as a longitudinally oriented channel or groove or concave recess or flattened portion on the front portion (52).
25. The storing component (50) according to embodiment 23, wherein the at least one connection element (53, 54) is configured as circumferential recess or groove on the front portion (52).
26. The storing component (50) according to any of the embodiments 23 to 25, wherein the front portion (52) comprises a first mating surface (53) and a second mating surface (54), the first mating surface (53) and the second mating surface (54) being configured and dimensioned for providing a removable connection by coupling with a respective first protrusion (28) and a second protrusion (29) provided in the cavity (21) of the applicator tip (20).
27. The storing component (50) according to embodiment 26, wherein the first mating surface (53) is configured and dimensioned for coupling to the first protrusion (28) by means of a transition fit
28. The storing component (50) according to embodiment 26 or 27, wherein the second mating surface (54) is configured and dimensioned are for coupling to the second protrusion (29) by means of an interference fit.
29. The storing component (50) according to embodiment 26 or 27 or 28, wherein the front portion (52) comprises a valve (30) configured to fluid-tightly couple with the first end (25a) of a cannula (25) of the applicator tip (20).
30. The storing component (50) according to embodiment 29, wherein the valve (30) comprises a membrane (31) which is impermeable to the fluid material inside the container (51), the membrane (31) comprising an elastic opening (32, 33, 34) for allowing the first end (28a) of the cannula (25) to be inserted in the container (51).
31. The storing component (50) according to embodiment 30, wherein the opening comprises at least a first cut (32) provided through the membrane (31).
32. The storing component (50) according to embodiment 31, wherein the opening comprises at least a second cut (33, 34) provided through the membrane (31), the first and the second cut crossing each other at a point of intersection (35) and being angularly offset with respect to one another about an axis of the storing component (50).
33. The storing component (50) according to any of the embodiments 30 to 32, wherein the membrane (31) is convex towards an inside of the container (51).
34. The storing component (50) according to any of the embodiments 30 to 33, wherein the front portion (52) includes an orifice (56) and an inner duct (57) for connecting the container (51) to the orifice (56).
35. The storing component (50) according to embodiment 34, wherein the inner duct (57) of the front portion (52) is divergent from the orifice (56) to the container (51).
36. The storing component (50) according to embodiment 35, wherein the inner duct (57) is inclined of an angle comprised between 1° and 3° with respect to an axis of the inner duct (57).
37. The storing component (50) according to any of the embodiments 23 to 36, further comprising a piston (60) movable in the container (51) for dispensing the fluid material, the piston (60) comprising at least a sealing lip (61) for providing a seal between the piston and the container, the piston being integrally formed in one piece with the sealing lip (61).
38. The storing component (50) according to embodiment 37, wherein the piston (60) comprises a front end having a piston axial protrusion (62) at least partially insertable in the inner duct (57).
39. The storing component (50) according to any of the embodiments 23 to 38, further comprising:
   an applicator closure tip (40) including:
      a cavity (41) defining a housing for the front portion (52),
      a rear end (42) comprising an opening (43) for allowing the front portion (52) to be inserted into the cavity (41),
      a front end (44) comprising a closed front wall (45) defining a bottom of the cavity,
      a side wall (46) extending between the rear end (42) and the front end (44),
   wherein the storing component (50) is configurable in at least a first configuration and a second configuration, and
   wherein in the first configuration the front portion (52) is outside the cavity (41) and the front portion (52) is attached to the applicator closure tip (40)
   by means of a breakable connection (47) for providing a closure to the orifice (56), and
   wherein in the second configuration the front portion (52) is housed in the cavity (41) of the applicator closure tip (40), at least one removable connection being provided between a first connection member (53) on the front portion (52) and a second connection member (48) on the side wall (46) of the closure tip (40), a fluid - tight contact being provided between
   the closed front wall (45) of the closure tip (40) and the front portion (52) for closing the orifice (56).
40. The storing component (50) according to embodiment 39, wherein the closed front wall (45) of the cavity (41) of the the applicator closure tip (40) comprises a front protrusion (49) for closing the orifice (56) in the second configuration.
41. The storing component (50) according to embodiment 40, wherein the front protrusion (49) is conically shaped.

### List of reference signs

- 10: applicator
- 20: applicator tip
- 21: cavity
- 22: rear end
- 23: opening
- 24: front end
- 25: cannula
- 25a: first cannula end
- 25b: second cannula end
- 26: side wall
- 27: inner surface
- 28: first protrusion
- 29: second protrusion
- 30: valve
- 31: membrane
- 32: elastic opening
- 33: elastic opening
- 34: elastic opening
- 36: ring
- 37: ring
- 40: applicator closure tip
- 41: cavity
- 42: rear end
- 43: opening
- 44: front end
- 45: front wall
- 46: side wall
- 47: breakable connection

- 48: second connection member
- 49: front protrusion
- 50: storing component
- 51: container
- 52: front portion
- 53: first mating surface
- 54: second mating surface
- 55: edge
- 56: orifice
- 57: duct
- 59: rear portion
- 60: piston
- 61: sealing lip
- 62: piston axial protrusion
- 63: gasket

- Y: longitudinal axis

## Claims

1. An applicator (10) including an applicator tip (20) and a storing component (50), the applicator tip (20) comprising:
a cavity (21) defining a housing for the storing component (50),
a rear end (22) comprising an opening (23) for allowing the storing component (50) to be partially inserted into the cavity (21),
a front end (24) defining a bottom of the cavity (21),
a side wall (26) extending between the rear end (22) and the front end (24), and
a cannula (25) extending through the front end (24) between a first cannula end (25a) and a second cannula end (25b), the first cannula end (25a) being located in the cavity (21);
the storing component (50) comprising:
a container (51) for storing a fluid material and
a front portion (52) configured to be housed in the cavity (21) of the applicator tip (20),
wherein
a removable connection is provided between the cavity (21) and the front portion (52) of the storing component (50), the removable connection comprising at least an interference fit or a snap-fit, and
a fluid-tight connection is provided between the cannula (25) and the storing component (50) for fluidically coupling the cannula (25) to the container (51).

2. The applicator (10) according to claim 1, wherein the removable connection comprises at least a first protrusion (28, 29) on one of the side wall (26) of the applicator tip (20) and the front portion (52) of the storing component (50), and the removable connection comprises at least a first mating surface (53, 54) on the other of the side wall (26) of the applicator tip (20) and the front portion (52) of the storing component (50), and wherein
the first protrusion (28, 29) and the first mating surface (53, 54) are configured and dimensioned for coupling to each other when the front portion (52) of the storing component is housed in the cavity (21).

3. The applicator (10) according to claim 2, wherein the removable connection comprises the first protrusion (28) and at least a second protrusion (29) on the side wall (26) and the removable connection comprises the first mating surface (53) and at least a second mating surface (54) on the front portion (52) of the storing component (50), the second protrusion (29) and the second mating surface (54) being configured and dimensioned for coupling to each other when the front portion (52) of the storing component is housed in the cavity (21), and
wherein the first protrusion (28) is closer to the front end (24) than the second protrusion (29).

4. The applicator (10) according to claim 3, wherein the first protrusion (28) and the first mating surface (53) are configured and dimensioned for coupling to each other by means of a transition fit and the second protrusion (29) and the second mating surface (54) are configured and dimensioned are configured and dimensioned for coupling to each other by means of an interference fit.

5. The applicator (10) according to claim 4, wherein the second mating surface (54) comprises an edge (55) protruding from the second mating surface and interposed between the second protrusion (29) and the first protrusion (28) when the front portion (52) of the storing component is housed in the cavity (21) with the first protrusion (28) and the second protrusion (29) respectively coupled to the first mating surface (53) and the second mating surface (54).

6. The applicator (10) according to any of the previous claims, wherein the front portion (52) comprises a valve (30) fluid-tightly coupled with the first end (25a) of the cannula (25) of the applicator tip when the front portion (52) of the storing component is housed in the cavity (21).

7. The applicator (10) according to claim 6, wherein the valve (30) comprises a membrane (31) which is impermeable to the fluid material inside the container (51), the membrane (31) comprising an elastic opening (32) for allowing the first end (25a) of the cannula (25) to be inserted in the container (51) when the front portion (52) of the storing component (50) is housed in the applicator tip (20).

8. The applicator (10) according to claim 1, wherein the front portion (52) includes an orifice (56) and an inner duct (57) for connecting the container (51) to the orifice (56), a fluid-tight connection being provided between the cannula (25) and the inner duct (57).

9. The applicator (10) according to claim 8, wherein the first cannula end (25a) comprising an external chamfer contacting the orifice (56) to provide said fluid - tight coupling.

10. The applicator (10) according to claim 9, wherein the cannula (25) is partially inserted in the inner duct (57) of the front portion (52), the cannula (25) contacting with the inner duct (57) to provide said fluid - tight connection.

11. An applicator tip (20) for an applicator (10), the applicator tip (10) comprising:
a cavity (21) defining a housing for the storing component (50) of the applicator (10),
a rear end (22) comprising an opening (23) for allowing the storing component (50) to be partially inserted into the cavity (21),
a front end (24) defining a bottom of the cavity (21),
a side wall (26) extending between the rear end (22) and the front end (24), the side wall (26) comprising an inner surface delimiting the cavity (21), and
a cannula (25) extending through the front end (24) between a first cannula end (25a) and a second cannula end (25b), the first cannula end (25a) being located in the cavity (21);
wherein,
the side wall (26) comprises at least one connection element (28, 29) for providing a removable connection between the cavity (21) and the front portion (52) of the storing component (50), said removable connection comprising at least an interference fit or a snap-fit.

12. A storing component (50) for an applicator (10), the storing component comprising:
a container (51) for storing a fluid material, and a front portion (52) configured to be housed in a cavity (21) of an applicator tip (20) of the applicator (10),
wherein the front portion (52) comprises
at least one connection element (53, 54) for providing a removable connection between the front portion (52) and the cavity (21) of the applicator tip (20), said removable connection comprising at least an interference fit or a snap-fit, and
a fluid-tight element (30, 57) for providing a fluid-tight connection between a cannula (25) of the applicator tip (20) and the storing component (50) for fluidically coupling the cannula (25) to the container (51).

13. The storing component (50) according to claim 12, wherein the storing component (50) extends from the front portion (52) to a rear portion (59) along a longitudinal axis (Y), and wherein the at least one connection element (53, 54) is configured as a longitudinally oriented channel or groove or concave recess or flattened portion on the front portion (52).

14. The storing component (50) according to any of the claims 12 or 13, further comprising:
an applicator closure tip (40) including:
a cavity (41) defining a housing for the front portion (52),
a rear end (42) comprising an opening (43) for allowing the front portion (52) to be inserted into the cavity (41),
a front end (44) comprising a closed front wall (45) defining a bottom of the cavity,
a side wall (46) extending between the rear end (42) and the front
end (44),
wherein the storing component (50) is configurable in at least a first configuration and a second configuration, and
wherein in the first configuration the front portion (52) is outside the cavity (41) and the front portion (52) is attached to the applicator closure tip (40) by means of a breakable connection (47) for providing a closure to the orifice (56), and
wherein in the second configuration the front portion (52) is housed in the cavity (41) of the applicator closure tip (40), at least one removable connection being provided between a first connection member (53) on the front portion (52) and a second connection member (48) on the side wall (46) of the closure tip (40), a fluid - tight contact being provided between the closed front wall (45) of the closure tip (40) and the front portion (52) for closing the orifice (56).

15. The storing component (50) according to claim 14, wherein the closed front wall (45) of the cavity (41) of the the applicator closure tip (40) comprises a front protrusion (49) for closing the orifice (56) in the second configuration.
